# EUROPEAN PATENT APPLICATION

(11) **EP 4 821 852 A1**
(43) Date of publication of application: **14.10.2026**
(21) Application number: 25169452.7
(22) Date of filing: 09.04.2025
(51) Int. Cl.: B65H 5/22, A61F 13/15, D04H 1/4258

(54) **PROCESS AND EQUIPMENT FOR SEPARATING SECTIONS OF A CONTINUOUS FIBROUS WEB**

(71) Applicant: COAX Technologies S.r.l., 26010 Monte Cremasco Cremona (IT)
(72) Inventor: ZAMPOLLO, Fabio, 26013 Crema (IT)
(74) Representative: Plischke, Manfred

(57) **Abstract**

The present invention is an apparatus for and the process of manufacturing a series of separated fibrous sections from a continuous fibrous web of the air-laying type, whereby the fibrous sections exhibit well defined cross-directionally extending margins. Such structures may suitably be absorbent, optionally comprising particulate material, and may be used for or as bed-pads or for other absorbent articles.

## Description

### Field of the invention

The present invention relates to an apparatus for and the process of manufacturing a series of separated fibrous sections from a continuous fibrous web of the air-laying type, whereby the fibrous sections exhibit well defined cross-directionally extending margins. Such structures may suitably be absorbent, optionally comprising particulate material, and may be used for or as bed-pads or for other absorbent articles.

### Background

Fibrous sheet materials, particularly fibrous sheet materials for absorbing fluids, are manufactured for many uses, for example they are incorporated into absorbent articles such as disposable diapers, incontinent bed-pads and catamenial napkins as fluid absorption or fluid transmission and/or diffusion elements, for example, as absorbent cores that are intended to absorb and retain body fluids.

Dry laying and, more specifically, air laying processes are widely used to produce webs from dry fibers, which can in turn be used e.g., as sheet materials for absorbing fluids. Particularly, the air laying process refers to the formation of webs with a random fiber orientation. The fibrous sheet materials produced by air laying processes are soft, flexible and porous, and are particularly suitable for use as liquid absorbent structures in absorbent articles, such as disposable diapers, sanitary napkins, pantiliners, incontinent or bed-pads, and wipes. Air laying processes are well known in the art, as are improvements aiming at evenly distributing the fibers in the airlaid structure and/or enhancing processability.

In US5527171 (Niro) the deposition of air suspended fibers in homogeneous layers is described by using multiple rows of rotating impellers in a forming head.

Similarly, US6233787 (Dan-Web / Advanced Nonwoven S/A) shows an apparatus for uniformly distributing a disintegrated material on a fiber layer forming surface by a stirrer having impellers rotating a short distance above a collecting surface.

US20030070262 (Oerlikon) describes two air-lay stations placed one after the other serving for the dry production of a non-woven fiber web, the stations including a fiber feed duct, an air laying forming head, a perforated screen as collecting surface, and a suction box for supporting the deposition of the fibers on the screen. A separate fiber source is part of each station.

Further, WO200004232 (M&J) describes the production of a nonwoven web of fibers out of a fibrous material, such as cellulose pulp. The fibrous material is disintegrated by a hammer mill and deposited at the outlet of a forming head on an endless collection wire, thereby forming a web of fibers. Nits are extracted from the forming head via a transport fan and a second air duct. Furthermore, the nits are defibrated and returned to the forming head.

Also, WO2004106604A1 (Oerlikon) aims at avoiding nits by employing a fiber distributor in a forming head at optimizing the rotational speed of the wings of the distributor.

Such production units are typically operated in an "off-line" modus, i.e., the webs produced thereon are prepared at a wide web width for interim storage in roll- or spool-form, or boxed ("festooned") and further transported to a converting place where these are fed into a process for forming articles, such as wipes, bed-pads and the like, including the cutting to size.

Unpublished co-pending application EP24188478 (CoAx) describes an approach for forming separated web sections by depositing fibers and/or particulate material onto a collection system, such as a collection belt, comprising a plurality of air permeable regions and blocked regions which are essentially non-permeable to air.

Whilst that provides separated fibrous sheets, the present inventor identified as an area for improvement that the cross-directionally extending margins of the separate sheets are not exhibiting a sharply defined cross-directionally extending margin. Such a somewhat fuzzy edge results in difficulties for further downstream process steps but also in deteriorated visual appearance.

Henceforth, the present invention aims at providing a series of web pieces or sections, that exhibit well and sharply defined cross-directionally extending edges.

### Summary

In a first aspect, the present invention is a web sections forming apparatus for creating a series of fibrous web sections from a continuous fibrous web, preferably a liquid absorbent fibrous web, which may optionally comprise particulate material, preferably superabsorbent polymer.

The apparatus comprises:
- a web support unit, adapted to have a web support surface moving machine-directionally from an upstream portion towards a downstream portion, and exhibiting a machine direction and a cross-machine direction and a height perpendicular thereto;
- a web forming unit for forming a continuous fibrous web, preferably comprising a fiber lay down forming box and optionally a particle addition unit,
- a fiber removal unit comprising
   o a fiber removal unit housing for separating a suction space of the fiber removal unit from the ambient air;
   o an air suction unit adapted to create a vacuum suction in the suction space of the fiber removal unit housing;
   o at least two, preferably at least four suction shoes, each being connected at its proximal end to the suction space and comprising at its distal end opposite to the proximal end a fiber removal opening extending at least 90 % of the cross-directional width of the web support surface,
   wherein the fiber removal opening is preferably surrounded by a suction shoe skirt, optionally elastic;
   o a suction shoe drive for moving at least the removal opening at a predetermined machine directional position in contact with or in proximity to the web.

Preferably, the fiber removal unit further comprises a removal opening distance adjustment tool selected from the group consisting of
- a suction shoe length adjustment tool,
- and a suction unit height adjustment tool.

In another aspect, the present invention is a method for creating a series of fibrous web sections from a continuous fibrous web, preferably a liquid absorbent fibrous web, which may optionally comprise particulate material, preferably superabsorbent polymer particles.

The method comprises the steps of
a) providing
   - a web sections forming apparatus as described in the above;
   - fibrous material and optionally particulate material;
b) forming a continuous fibrous web from the fibrous material, optionally comprising particulate material in the web forming unit on the web support surface of the web support unit;
c) moving the fibrous web downstream towards the fiber removal unit at a web speed, preferably without a bonding step;
d) positioning at least one fiber removal opening of a suction shoe of the fiber removal unit by the suction shoe drive at a predetermined machine directional position in contact with or in proximity to the web;
e) applying an air suction by the air suction unit to the suction shoe adapted to remove essentially all fibers and particles, if present, of the web through the fiber removal opening, thereby creating an essentially fiber/particle free section at a predetermined machine-directional extension between fibrous sections;
f) positioning a further fiber removal opening of a further suction shoe at a predetermined position upstream of the fiber/particle free section and applying air suction, thereby creating a further fiber/particle free section.

Steps d) to f) are repeated in this order so as to form a series of fibrous web sections separated by fiber/particle free sections.

Thereby, the suction shoe drive is adapted to move the suction shoe opening at a machine-directionally speed synchronized with the web speed to more than about 90 % and less than about 110%, preferably at 100% of the web speed,
- by rotating the at least two suction shoes around a suction shoe axis, optionally concurrently reciprocating the suction shoe axis machine directionally,
   or
- by moving a multiplicity, preferably at least six suction shoes along a closed loop path, preferably an oval shaped path, such that the suction openings of at least two suction shoes are concurrently moving parallel to and in contact with or in proximity to the fibrous web, thereby creating fiber/particle free sections between fibrous web sections.

Optionally, in step c) of applying an air suction by the air suction unit to the suction shoe the air suction may be applied to the suction shoe only when the suction shoe is in a suction position. Optionally, the removed fibers and particles, if present, may be recycled, preferably to the web forming unit.

### Brief description of the Figures

Fig. 1 A to C show an apparatus according to the present invention and particular features thereof.
Fig. 2 shows another execution of an apparatus according to the present invention.
Fig. 3 A and B show further executions of an apparatus according to the present invention.
Fig. 4 shows an even further set-up for an apparatus according to the present invention.

The figures are schematic only, and not to scale. Same numerals refer to same or equivalent features or elements, single (') or multiple (", ‴, ...) apostrophes indicate duplicate features, such a left and right or front and back, etc. Omitting features in one figure does not imply that these cannot be combined with features of other figures.

### Detailed description

In a first aspect, the present invention relates to an apparatus and in a second aspect to the process for separating fibrous structures comprising short fibers, such as cellulosic fibers, from a continuous fibrous web, optionally comprising particulate material.

Wood pulp fibers are a preferred starting material as may be formed by a variety of pulping processes, such as kraft pulp, sulfite pulp, thermomechanical pulp, and the like. Further, the wood fibers may be any high-average fiber length wood pulp, low-average fiber length wood pulp, or mixtures of the same. One example of suitable high-average length wood pulp fibers includes softwood fibers such as, but not limited to, northern softwood, southern softwood, redwood, red cedar, hemlock, pine (e.g., southern pines), spruce (e.g., black spruce), combinations thereof, and the like. One example of suitable low-average length wood pulp fibers includes hardwood fibers, such as, but not limited to, eucalyptus, maple, birch, aspen, and the like. The fibers may be treated so as to allow optimization of processing and or absorbency properties, The term "treated" as used herein is understood to include any means of introducing the additive to the fiber and/or fibrous matrix, but not limited to, such as coating, spraying, printing, chemical modifications, wet-end additions applications to the fibers as well as blending untreated fibers with treated fibers.

Moreover, if desired, secondary fibers obtained from recycled materials may be used, such as fiber pulp from sources such as, for example, newsprint, reclaimed paperboard, and office waste, or recycled diapers, be it from factory scrap or be it post-consumer recycling.

The fibrous material may be delivered as fibrous board or in fibrous sheet form. Also bales of fibrous material may be delivered as may be opened prior to be used in the process according to the present invention. Further, "roughly graded material" may be employed, wherein fibers are present as clusters of several hundred up to several thousand fibers in the roughly graded material. It is an object of the present invention to dry lay, or air lay, such fibrous material to form fibrous webs for being converted into articles comprising such fibrous webs, such as absorbent articles, such as wipes, or pads like bed-pads, or the like. Such articles comprise further materials, typically web materials for stabilizing and/or containing the fibrous webs.

The webs may further comprise particulate material, such as superabsorbent polymeric (SAP) particles, as well known for absorbent articles, as may be referred to as "fiber/particle" webs. Conventional air-laying processes for forming such webs are often operated in an "off-line" modus, whereby jumbo reels of the fiber/particle structure are produced on machines with a large width. Such wide roll may be cut to a lower width and rewound for being transferred to a converter, where the webs are cut to the appropriate size for being introduced into articles.

Whilst such processes allow efficient production of the fiber/particle webs, rewinding and transport imply process complexity and cost. When avoiding this by producing airlaid webs in-line on the converting process, conventional air-laying processes provide continuous webs that imply potential for contamination when being separated to the right length, and further require cut and space operation to be fed into the finished articles.

Thus, in the first aspect, the present invention is an apparatus for creating a series of separated fibrous web sections for being processed in further processing steps for forming articles comprising fibrous structures, optionally with particulate material.

In another aspect, the present invention is a process for converting a continuous airlaid fibrous web into a series of fibrous sections, whereby fibrous sections are separated by regions which are free of the airlaid fibrous material and of the particulate material.

In order to further explain the apparatus of and the method for executing the present invention, reference is made to the figures, which however, should not be seen to limit the present invention. In particular features as explained with reference to one figure may also be employed in the context of other figures.

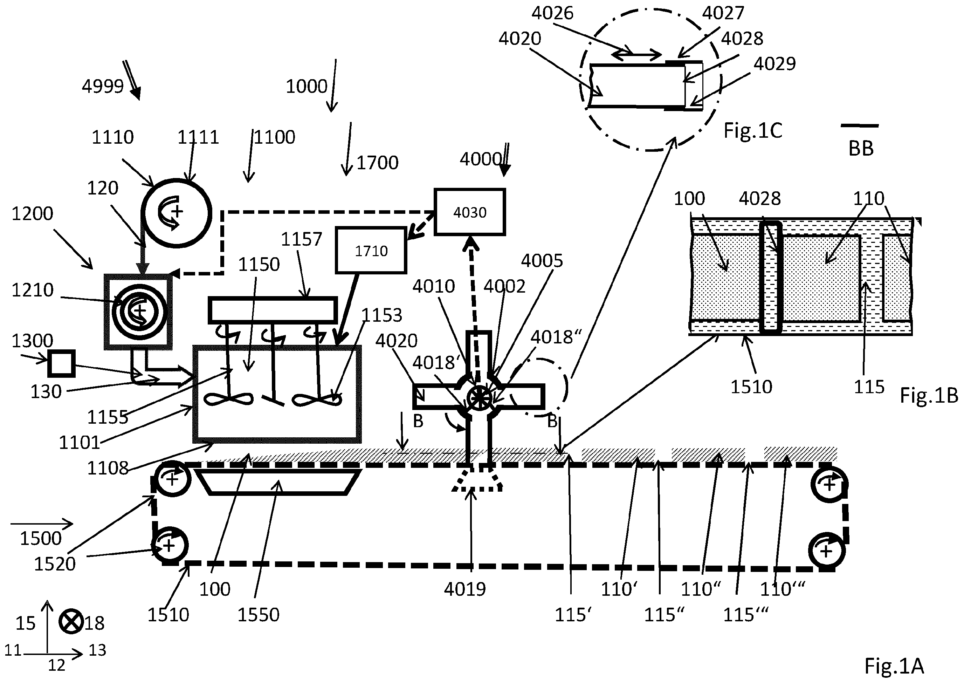

As shown in Fig. 1, the web sections forming apparatus 4999 comprises exemplarily a well-known equipment 1000 for forming a fibrous web, comprising a fiber supply 1110, e.g., an unwinder 1111 for delivering a fiber board 120 to a disintegration unit 1200, such as a hammer mill 1210. Alternatively or concurrently, the fibers may be delivered from other fiber supply means (not shown) such as when being delivered in bale form or from a silo or bin. Disintegrated fibers 130 are transferred, preferably pneumatically by means of air supply 1300, towards the forming box 1101 of a web forming unit 1100. As depicted, the web forming unit may comprise fiber homogenizer 1150, such as fans 1153 rotating by drive 1157 via vertical axes 1155. Optionally, and often preferably, particulate material, such as superabsorbent polymer (SAP) particles, may be fed by a particle application unit from a particle supply 1710 to the web forming unit, where these may be well intermixed with fibers, whilst these are cross-directionally 18 and machine-directionally 12 homogenized. The fibers, and optionally the particulate material, are further deposited through the forming box outlet 1108 onto a collection system 1500, forming an essentially unbonded continuous web 100, as may be also referred to as "fiber/particle" web.

In Fig. 1, the collection system 1500 is depicted as a belt collector unit comprising a foraminous belt 1510 driven and guided by dive and guide rolls 1520. The collecting is further supported by a suction system 1550. The foraminous belt and the fibrous web deposited there on are moving in the machine direction 12, transferring the web from the upstream region 11 towards the downstream 13 region of the equipment.

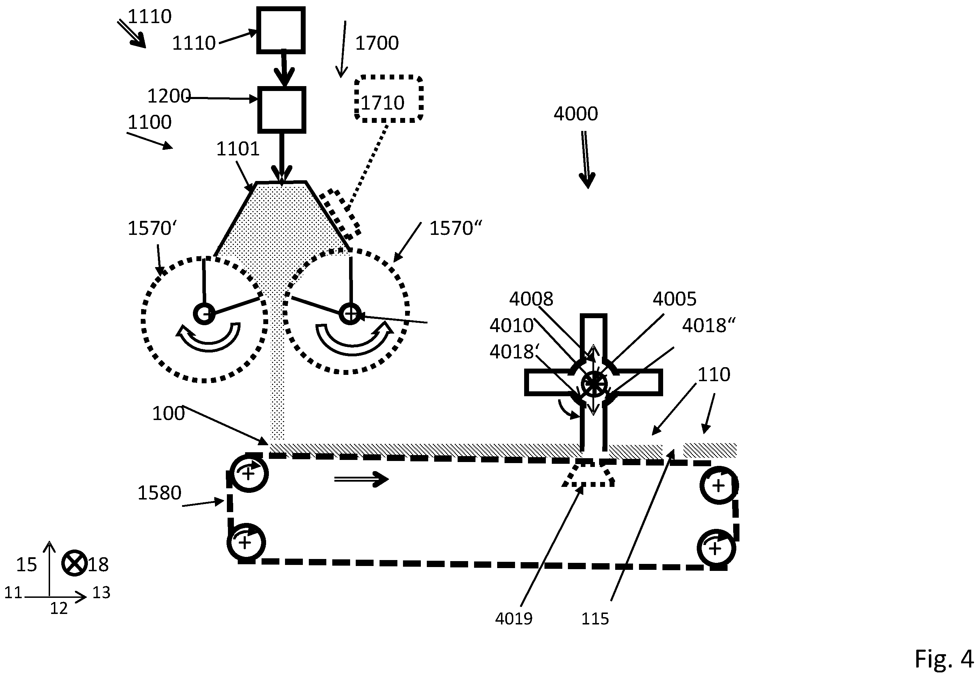

As indicated in Fig. 4, instead of a belt collector unit, the collection system may comprise two counterrotating vacuum drums 1570', 1570" for receiving the fibers and optionally particles from a particle supply 1710, whereby a slightly compressed but still essentially unbonded web 100 is deposited on a web transfer system, such as a transfer belt 1580.

Referring again also to Fig. 1 A to C, the present invention employs a fiber removal unit 4000 for selectively removing fibers and particles, if present, in predetermined regions, so as to create fiber / particle free regions 115 between subsequent web sections 110. To this end, a first suction shoe 4020' is positioned z-directionally 15 over the web 100 in predetermined regions. Upon application of air suction from an air suction unit 4010, the fibers / particles are removed through a removal shoe opening 4028 from the web with a sharp edge definition of the web/particle sections 110. Preferably, the fibers and particles may be separated in a separation and recycling unit 4030, such as in a filtering unit, for being recycled to the fiber (1210) and/or particle supply (1710) or directly to the web forming unit 1000 (not shown).

Fig. 1A shows exemplarily a first execution of a fiber removal system comprising four suction shoes. However, this number can be varied and even a less preferred execution with a single suction shoe rotating is conceivable, which - for comparable size of the web sections 110 and fiber/particle free regions 115 - rotates at a higher speed than for multiple suction shoes.

A suction shoe opening 4028' of a removal shoe 4020' travels with a machine directional speed corresponding to the web speed or deviates no more than about plus or minus 10% thereof, thereby increasing the fiber / particle free region accordingly. Whilst other shapes are contemplated, the suction shoe opening exhibits preferably a rectangular MD/CD shape, optionally with rounded corners, as indicated in the Fig. 1B showing a cross-sectional view BB in Fig. 1A, and covers preferably the cross-directionally 18 the full width of the web, but at least 90 % or at least 95 % thereof.

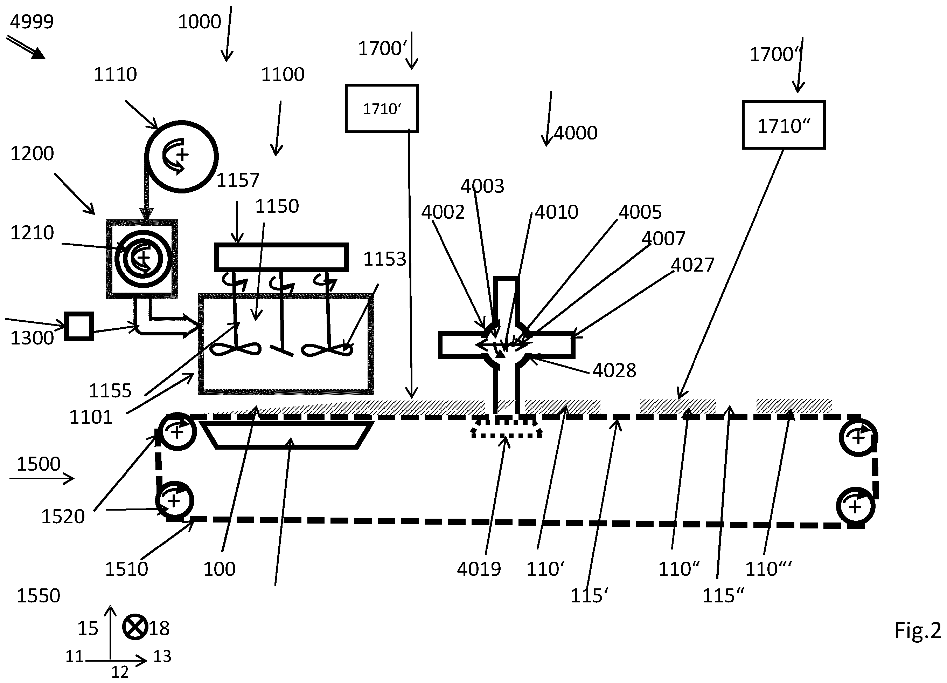

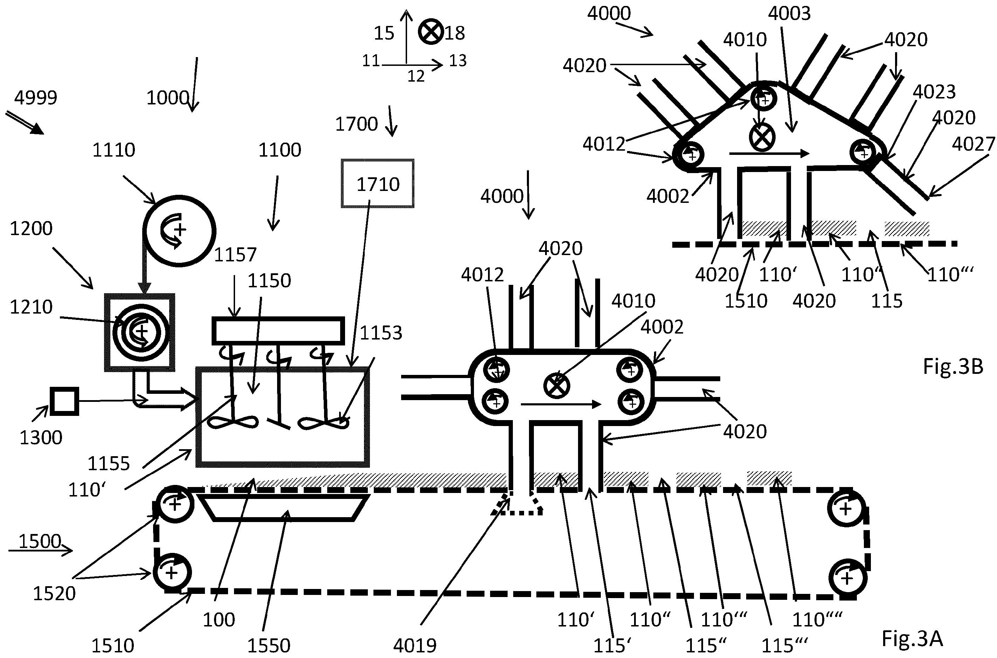

The movement of the suction shoe may be achieved by a variety of means, such as depicted in Fig. 1 A to C and 4, but also Fig. 2 and Fig. 3. As shown in Fig. 1A, multiple suction shoes 4020 rotate around a removal unit axis 4005 such that a suction shoe opening is positioned over the web or is immersed into the web to then remove the fiber / particles in this particular region. With the rotational speed synchronized to - or only slightly deviating from - the web speed, the appropriate fiber / particle free regions are formed and the suction shoe 4020' rotates further away from the web, whilst a subsequent suction shoe 4020" approaches the web, and so on. Thus, the length of the fiber / particle free region is defined by the MD extension of the suction shoe opening and - if present - the speed deviation between suction shoe movement and web speed.

The machine directional extension of the web sections 110 can be adjusted by the distance of the rotating axis to the web respectively web support and the corresponding radial extension of the suction shoe.

Referring to Fig. 1C, the suction shoe opening at the distal end 4027 may optionally be circumscribed by a suction shoe skirt 4029, as may be flexible so as to allow tight tolerances between suction shoe 4020 and foraminous web support 1510 carrying the web 100.

Optionally, the removal of the fibers / particles may be eased by applying an air blow through an air blowing nozzle 4019 from the opposite side of the web support 1015. This may provide a short intermittent air blow so as to initiate the detachment of the fibers / particles from the collection system 1015. It may also provide a series of blows from several nozzles arranged machine directionally or move translatory or reciprocating so as to cover the fiber / particle removal period. As also depicted in Fig. 1B, such a set-up and its operation allow a sharp CD edge definition of the web sections 110.

As shown in Fig. 1A, the fiber removal shoes 4020 may be connected to a fiber removal unit housing 4002, or may be connected to each other so as to form the housing. Preferably, air flow baffles 4018 allow optimization of the sir suction flow by having only the "active" suction shoe 4020 being connected to the air suction system.

Fig. 2B depicts a variant corresponding to the one as explained in the context of Fig. 1, wherein further the axis 4005 of the rotating fiber / particle removal unit 4000 reciprocates machine directionally 12 by a reciprocating drive 4007. Depending on the speed of this movement relative to the web speed, such an additional movement can be employed to either increase the suction period for a more complete removal of fibers / particles, or to increase the MD extension of the fiber / particle free region. Also included in Fig. 2 are further options for the particulate material addition system 1700, namely feeding the particulate material from a particulate material supply 1710', 1710" onto the surface of the formed web 100 or onto the surface of the separated web sections 110, respectively.

Fig. 3 depicts yet another approach for the arrangement of the suction unit 4000, wherein a multiplicity of suction shoes 4020 is connected to a closed loop suction shoe belt system 4012, whereby the belt system may form an oval shape, also referred to as "stadium" shape, i.e., a rectangle with rounded corners, as depicted in Fig. 3A, or a triangular shape, as in Fig. 3B, or any other configuration where at least a portion thereof can be operated parallel to the web 100, preferably at matched speed with the web 100. The suction shoe belt system 4012 may form an airtight vacuum housing 4002 or be positioned within an airtight housing 4002 confining a suction space 4003. Thereby, a lower section of the shoe belt system is positioned towards the web 100 on the web support 1510. The fiber removal openings 4028 of the suction shoes 4020 correspond to the machine-directional extension of the fiber/particle free section 115 between web sections 110, and the machine-directional spacing between two adjacent shoes 4020 corresponds to the machine-directional extension of the fibrous web sections 110.

For any of the above executions, the suction as well as the air blowing nozzle may be operated intermittently, optionally supported by air baffles 4018 air suction housing 4002 or in the suction shoes 4020.

Optionally, the fiber removal unit 4000 may be designed with a height adjusting tool 4008, so as to avoid change parts in case of a size change of the articles via the MD-extension of the web sections 110.

## Claims

1. A web sections forming apparatus (4999) for creating a series of fibrous web sections (110) from a continuous fibrous web (100),
preferably a liquid absorbent fibrous web,
optionally comprising particulate material, preferably superabsorbent polymer,
said apparatus comprising
- a web support unit (1500),
adapted to have a web support surface (1510) moving machine-directionally from an upstream portion (11) towards a downstream portion (13),
and exhibiting a machine direction (12) and a cross-machine direction (18) and a height (15) perpendicular thereto;
- a web forming unit (1100) for forming a continuous fibrous web,
preferably comprising a fiber lay down forming box (1101);
and optionally a particle addition unit (1710);
- a fiber removal unit (4000) comprising
∘ a fiber removal unit housing (4002) for separating a suction space (4003) of said fiber removal unit from the ambient air;
o an air suction unit (4010) adapted to create a vacuum suction in said suction space (4003) of said fiber removal unit housing (4002);
o at least two, preferably at least four suction shoes (4020),
each being connected at its proximal end (4023) to said suction space (4002) and comprising at its distal end (4027) opposite to said proximal end (4023) a fiber removal opening (4028) extending at least 90 % of the cross-directional width (18) of said web support surface (1510),
wherein said fiber removal opening (4028) is preferably surrounded by a suction shoe skirt (4029), optionally elastic;
o a suction shoe drive (4025) for moving at least said removal opening (4028) at a predetermined machine directional position in contact with or in proximity to said web (100).

2. A web sections forming apparatus (4999) according to claim 1, wherein said fiber removal unit (4000) further comprises a removal opening distance adjustment tool selected from the group consisting of
- a suction shoe length adjustment tool (4026),
- and a suction unit height adjustment tool (4008).

3. A method for creating a series of fibrous web sections (110)
from a continuous fibrous web (100),
preferably a liquid absorbent fibrous web,
optionally comprising particulate material, preferably superabsorbent polymer particles,
said method comprising the steps of
a) providing
- a web sections forming apparatus (4999) according to any of claims 1 or 2;
- fibrous material and optionally particulate material;
b) forming a continuous fibrous web (100) from said fibrous material, optionally comprising particulate material,
in said web forming unit (1101)
on said web support surface (1510) of said web support unit (1500);
c) moving said fibrous web (100) downstream (13) towards said fiber removal unit (4000) at a web speed,
preferably without a bonding step;
d) positioning at least one fiber removal opening (4028') of a suction shoe (4020') of said fiber removal unit (4000) by said suction shoe drive (4025) at a predetermined machine directional position in contact with or in proximity to said web (100);
e) applying an air suction by said air suction unit (4010) to said suction shoe (4020') adapted to remove essentially all fibers and particles, if present, of said web (100) through said fiber removal opening (4028),
thereby creating an essentially fiber/particle free section (115') at a predetermined machine-directional (12) extension between fibrous sections (110);
f) positioning a further fiber removal opening (4028") of a further suction shoe (4020") at a predetermined position upstream (11) of said fiber/particle free section (115') and applying air suction,
thereby creating a further fiber/particle free section (115");
repeating steps d) to f) in this order so as to form a series of fibrous web sections (110) separated by fiber/particle free sections (115),
thereby
moving said suction shoe opening (4028) by said suction shoe drive (4025) at a machine-directionally (12) speed synchronized with said web speed to more than about 90% and less than about 110%, preferably at 100% of said web speed,
- by rotating said at least two suction shoes (4020) around a suction shoe axis (4005),
o optionally concurrently reciprocating said suction shoe axis (4005) machine directionally,
or
- by moving a multiplicity, preferably at least six suction shoes (4020) along a closed loop path, preferably an oval shaped path, such that said suction openings (4028) of at least two suction shoes (4020) are concurrently moving parallel to and in contact with or in proximity to said fibrous web (4020).

4. A method for creating a series of fibrous web sections (110) according to claim 3,
wherein in step c) of applying an air suction by said air suction unit (4010) to said suction shoe (4020) said air suction is applied to said suction shoe (4018) only when said suction shoe (4018) is in a suction position.

5. A method for creating a series of fibrous web sections (110) according to claim 4 or 5, further comprising the step of recycling of fibers and particles, if present, preferably to said web forming unit.
